(19) Europäisches Patentamt
European Patent Office
Office européen des brevets

(11) Publication number: 0 008 525 A2

# (12) EUROPEAN PATENT APPLICATION

(21) Application number: 79301671.8

(22) Date of filing: 16.08.79

(51) Int. Cl.$^3$: A 61 K 9/00
A 61 K 31/43, A 61 K 31/545

(30) Priority: 25.08.78 GB 3461078

(43) Date of publication of application: 05.03.80 Bulletin 80/5

(84) Designated Contracting States: AT BE CH DE FR GB IT LU NL SE

(71) Applicant: BEECHAM GROUP LIMITED
Beecham House Great West Road
Brentford, Middlesex(GB)

(72) Inventor: Taskis, Charles Bernard
Hillside
Durrington Hill Worthing, Sussex(GB)

(72) Inventor: Greenway, Michael John
15 Church Way
Tarring Worthing, Sussex(GB)

(74) Representative: Dawson, Hugh Bainforde et al,
Beecham Pharmaceuticals Great Burgh Yew Tree
Bottom Road
Epsom Surrey KT18 5XQ(GB)

## (54) Pharmaceutical compositions and process for their preparation.

(57) Water/Ethanol/Polyethylene glycol or propylene glycol as a vehicle for injectable clavulanic acid salt/$\alpha$-amino penicillin and cephalosporin salt compositions gives a formulation of improved stability.

EP 0 008 525 A2

TITLE MODIFIED
see front page

PHARMACEUTICAL COMPOSITIONS

This invention relates to pharmaceutical compositions. More specifically this invention relates to anti-bacterial pharmaceutical compositions which maybe administered by injection.

U.K. Patent No. 1508977 described clavulanic acid, of formula (I) :

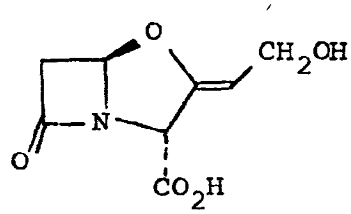

(I)

and its salts; and the use of such compounds as synergists for penicillins and cephalosporins.

The administration of such synergistic formulations by injection is also described, the injectable compositions being made up by the addition of water to the synergistic formulations.

It has been found that certain of these injectable compositions lose potency fairly quickly on reconstitution with water so that they have to be administered shortly after this reconstitution.

It is an object of this invention to provide an injectable pharmaceutical composition having improved stability relative to the hereinbefore described compositions.

This object is attained by the provision of a specific reconstitution mixture.

Accordingly, the present invention provides an injectable pharmaceutical composition, which composition comprises a water soluble salt of clavulanic acid and a water soluble salt of an α-amino penicillin or cephalosporin, and a vehicle of (a) water, (b) ethanol and (c) polyethylene glycol or propylene glycol.

In one particular useful aspect, the present invention provides an injectable pharmaceutical composition, which composition comprises a solution of a water soluble salt of clavulanic acid and a water soluble salt of an α-amino penicillin or cephalosporin in a vehicle of (a) water, (b) ethanol and (c) polyethylene glycol or propylene glycol.

The polyethylene glycol used in this invention may be any having an average molecular weight of less than 4000. (Higher molecular weight glycols have been found to increase the viscosity to an unacceptable level). It has been found that glycols in the molecular weight range 180-600, especially 200-400, are particularly acceptable. A single glycol or any mixture of glycols within this molecular weight range may be used.

With polyethylene glycol, the ratio of water to organic solvents present in the composition will normally be in the range of 80:20 to 40:60, usually 70:30 to 50:50 and more usually 65:35 to 55:45. [These ratios are volume/volume].

With polyethylene glycol, suitably the ratio of glycol to ethanol is 2:1 to 1:2, more suitably 1:1 [These ratios are volume/volume].

Particularly suitable ratios of water:ethanol: polyethylene glycol present in these compositions

approximate to 60:20:20.

Turning now to propylene glycol, the ratio of water to organic solvents present in the composition will normally be in the range 60:40 to 5:95, more suitably 50:50 to 20:80, for example 30:70. [These ratios are volume/volume]

Similarly, suitably the ratio of propylene glycol to ethanol in such compositions is 8:1 to 1:2, preferably 5:1. [These ratios are volume/volume]

Particularly suitable ratios of water:ethanol: propylene glycol are 40:20:40 and 30:10:60.

It will be appreciated that it is possible to use a mixture of polyethylene glycol and propylene glycol as the glycol component of the composition. However, it is greatly preferred that the two glycols are used separately.

The ratio of antibacterial agents to vehicle present in the composition is normally from 2:20 to 7:20, usually from 2:20 to 4:20, and generally approximates to 7:40. [These ratios are weight (taken as free acid equivalent weight)/volume].

The ratio of clavulanate salt to penicillin or cephalosporin salt present in the composition will normally be in the range 10:1 to 1:10, usually in the range 1:5 to 1:1, and will preferably be 1:2. [These ratios are weight/weight ratios based on free acid equivalent weights].

Suitably the clavulanate salt will be the sodium or potasium salt, preferably the potassium salt.

Suitably the penicillin or cephalosporin salt is the sodium or potassium salt, preferably the sodium salt.

α-Amino penicillins and cephalosporins have a side chain of structure (II):

$$R - \overset{D}{\underset{\underset{NH_2}{|}}{CH}} - CO - NH - \qquad (II)$$

(joined at the 6- or 7- position respectively).

A particularly useful class of such penicillins and cephalosporins has R in structure (II) being phenyl, p-hydroxyphenyl or cyclohexadienyl. Examples of such penicillins include ampicillin and amoxycillin, and of such cephalosporins include cephradine.

It has been found that this invention is particularly pertinent to formulations wherein the penicillin is amoxycillin.

Thus one preferred embodiment of this invention is an injectable pharmaceutical composition comprising a solution of sodium or potassium clavulanate and sodium or potassium amoxycillin in a vehicle of (a) water, (b) ethanol and (c) polyethylene glycol.

Another preferred embodiment of this invention is an injectable pharmaceutical composition comprising a solution of sodium or potassium clavulanate and sodium or potassium amoxycillin in a vehicle of (a) water, (b) ethanol and (c) propylene glycol.

In these preferred embodiments the relative proportions of the ingredients are suitably as hereinbefore.

Most preferably in these embodiments the active ingredients are potassium clavulanate and sodium amoxycillin.

In another aspect, the invention also provides a twin pack in which the first part of the pack contains

the active ingredients and the second part of the pack contains the vehicle.

The invention also provides a process for preparing the composition of the invention, which process comprises bringing into association the active ingredients and the vehicle.

The exact nature of this process of course varies with the nature of the compositions to be prepared.

Whn the composition is in solution form, then the process comprises dissolving the active ingredients (either simultaneously or consecutively) in the vehicle.

When the composition is in the twin pack form, then the process comprises filling the appropriate ingredients into separate parts of the pack.

The twin pack of this invention will generally comprise a vial containing the mixed active ingredients in the form of a dry powder and/or a freeze dried plug and a second vial containing the mixed solvents. In order to form the injectable solution the liquid from the second vial will be transferred to the first vial.

The vial containing the active ingredients is conveniently filled by freeze drying a solution of the clavulanate salt in situ and thereafter adding to the resulting plug the pencillin or cephalosporin salt in powder form. These dry components may then be sealed into a vial. Alternatively the vial may be filled in a convenient conventional manner with separately prepared dry salts in powder form.

The injectable compositions of this invention may be used in the manner described in U.K. Patent No. 1508977.

In yet a further aspect, the invention provides a method of improving the stability of an aqueous

injectable solution of a water soluble salt of clavulanic acid and a water soluble salt of an α-amino penicillin or cephalosporin, which method comprises incorporating in the solution ethanol and either polyethylene glycol or propylene glycol as hereinbefore described.

The following Examples illustrate the invention.

EXAMPLE 1

2.5 mls of a 5% solution of sodium clavulanate in water (calculated on pure free acid equivalent) was filled into a 5 ml vial. The solution was freeze dried over 24 hours with a final temperature of 40°C to yield a plug of sodium clavulanate at the bottom of the vial. To this was added 250 mg amoxycillin as a dry powder of sodium amoxycillin and the vial closed.

In order to produce an injectable solution, 1.5 ml of a mixture containing 60/20/20 of water/ethanol/polyethylene glycol (Mol. wt 200) was added. This solution may be injected using a 21 gauge needle.

The improved stability of this composition in comparison to a simple aqueous solution (1.5 mls of water) was shown by forming the solutions at 20°C and comparing the potencies at frequent intervals. It was found that 10% of the potency of the sodium clavulanate was lost in aqueous solution after only 8 minutes but 10% of the potency of the sodium clavulanate in the mixed solvents was not lost until 30 minutes.

This composition contained 250 mg. amoxycillin as free acid and 125 mg. clavulanic acid as free acid.

EXAMPLE 2

The following solutions may be prepared.

| | A | B | C | D | E | F |
|---|---|---|---|---|---|---|
| Sodium clavulanate[a] | 25 | 50 | 100 | 125 | 100 | 50 |
| Sodium amoxycillin[b] | 125 | 250 | 500 | 250 | 100 | 100 |
| Water[c] | 0.4 | 0.9 | 1.8 | 0.9 | 0.6 | 0.4 |
| Ethanol[c] | 0.15 | 0.3 | 0.6 | 0.3 | 0.2 | 0.15 |
| Polyethylene glycol[c] | 0.15 | 0.3 | 0.6 | 0.3 | 0.2 | 0.15 |

a Weight in mgs equivalent to free acid

b Weight in mgs equivalent to free acid

c Vol in ml; molecular weight of glycol 200-400

Similar solutions may be prepared by replacing the polyethylene glycol with the same amounts of propylene glycol or by the same amount of a 1:1 mixture of the polyethylene and propylene glycols.

EXAMPLE 3

The process of Example 1 was repeated but using potassium clavulanate in place of sodium clavulanate, a polyethylene glycol of molecular weight 400 in place of the polyethylene glycol of molecular weight 200, and a reconstitution volume of 2 ml instead of 1.5 ml.

A very similar improvement of stability was found with this injectable solution as with the solution of Example 1.

This composition contained 250 mg amoxycillin as free acid and 125 mg clavulanic acid as free acid.

EXAMPLE 4

The process of Example 3 was repeated but using sodium cephradine in place of sodium amoxycillin.

The improved stability of this composition in comparison to a simple aqueous solution (2 mls of water) was shown by forming the two solutions at 20$^{o}$C and comparing their potencies at frequent intervals. It was found that 10% of the potency of the potassium clavulanate was lost in aqueous solution after only 90 seconds, while in the mixed solvents this figure was only reached after 10 minutes.

This composition contained 250 mg cephradine as free acid and 125 mg clavulanic acid as free acid.

Example 5

Potassium clavulanate equivalent to 125 mg free acid and sodium amoxycillin equivalent to 250 mg free acid was dissolved in 2 ml of a solvent consisting of water/ethanol/propylene glycol in the proportions 40/20/40. The potency of the clavulanate component took 31 minutes to drop by 10%, compared with 10 minutes for a totally aqueous solution.

Example 6

For an identical solution to that of Example 5 but containing 250 mg f.a. amoxycillin as the sodium salt and 50 mg f.a. clavulanic acid as the potassium salt, the stability in solution was found to improve from 8 to 30 minutes for the clavulanic acid component for 10% loss of potency.

Example 7

Example 5 was repeated exactly, but using the different vehicles shown in the Table. The improved stability found with these formulations is shown in the Table.

TABLE

| Vehicle | | | Time (mins) 10% Potency Loss Clavulanate Acid Component |
|---|---|---|---|
| Propylene Glycol | Ethanol | Water | |
| 40 | 10 | 50 | 21 |
| 30 | 20 | 50 | 22 |
| 30 | 30 | 40 | 26 |
| 20 | 20 | 60 | 16 |
| 40 | 30 | 30 | 35 |
| 30 | 40 | 30 | 40 |

<u>What we claim is</u>:

1. An injectable pharmaceutical composition, which composition comprises a water soluble salt of clavulanic acid and a water soluble salt of an α-amino penicillin or cephalosporin, and a vehicle of (a) water, (b) ethanol and (c) polyethene glycol or propylene glycol.

2. A composition according to claim 1, which comprises a solution of a water soluble salt of clavulanic acid and a water soluble salt of an α-amino penicillin or cephalosporin in a vehicle of (a) water, (b) ethanol and (c) polyethylene glycol or propylene glycol.

3. A composition according to claim 1, in the form of a twin pack in which the first part of the pack contains the water soluble salt of clavulanic acid and the water soluble salt of an α-amino penicillin or cephalosporin, and the second part of the pack contains the vehicle of (a) water, (b) ethanol and (c) polyethylene glycol or propylene glycol.

4. A composition according to any one of the claims 1 to 3, wherein the α-amino penicillin is ampicillin or amoxycillin, or the α-amino cephalosporin is cephradine.

5. A composition according to claim 4, wherein the active ingredients are sodium or potassium clavulanate and sodium or potassium amoxycillin.

6. A composition according to any one of the preceding claims, wherein component (c) of the vehicle is polyethylene glycol in the molecular weight range 200-400.

7. A composition according to any one of the preceding claims wherein the component (c) of the vehicle is polyethylene glycol; and the ratio of water to organic solvents in the vehicle is 70:30 to 50:50; and the ratio of glycol to ethanol is 2:1 to 1:2.

8. A composition according to any one of the claims 1 to 5, wherein the component (c) of the vehicle is propylene glycol; and the ratio of water to organic solvents in the vehicle is 50:50 to 20:80; and the ratio of glycol to ethanol is 8:1 to 1:2.

9. A composition according to claim 1, 2 or 3, wherein the active ingredients are potassium clavulanate and sodium amoxycillin, and the vehicle is a 40:20:40 mixture of water, ethanol and propylene glycol.

10. A process for the preparation of composition according to any one of the claims 1 to 9, which process comprises bringing into association the active ingredients and the vehicle.